# EUROPÄISCHE PATENTANMELDUNG

(11) **EP 2 599 393 A1**
(43) Veröffentlichungstag der Anmeldung: **05.06.2013**
(21) Anmeldenummer: 12194602.4
(22) Anmeldetag: 28.11.2012
(51) Int. Cl.: A23L 1/302, A23L 1/304, A23L 1/30, A61K 35/12, A61P 43/00

(54) **Ölige Zubereitungen von und mit Lecithinen ohne Etherphospholipide für pflegende, diätetische, kosmetische Kompositionen oder Heilmittel für sensible Personen**

(30) Priorität: 29.11.2011 DE 202011108417 U
(71) Anmelder: Korth, Ruth-Maria, 80639 München (DE)
(72) Erfinder: Korth, Ruth-Maria, 80639 München (DE)
(74) Vertreter: Beck, Alexander

(57) **Zusammenfassung**

Ölige Zubereitung von und mit Lecithinen, bei der keine Etherphospholipide gebildet werden bei der Zubereitung von pflegenden, diätetischen Kompositionen und Heilmitteln, die angereichert werden ohne Bildung von Etherphospholipiden.

## Beschreibung

Die Erfindung betrifft die lokalen, dietätischen Zubereitungen von Kompositionen, die gekennzeichnet sind durch Ausschluss, Hemmung, Minderung von Etherlipiden, Etherphospholipiden, Alkylethanolaminen, Alkylglycerol, Alkylradikalen, Alkylglycerol-Derivaten, Plasmalogenen, deren Vorstufen und deren Derivate (folgend "Etherlipide, Alkyl-Derivate oder AAGPC").

Der Gegenstand der Erfindung betriff ölige Zubereitungen und neue Herstellungsverfahren für Kompositionen, die von Beginn keinen relevanten Gehalt von Etherphospholipiden enthalten und während der Zubereitung keien Etherpshopholipide bilden. Die Erfindung nimmt die Priorität vom Gebrauchsmuster 20 2011 108 in Anspruch. Das Gebrauchsmuster beweist die schädliche Wirkung von Alkoholmetaboliten und damit auch der alkoholabhängigen Etherphospholipide. Pflegende, dietätische, lokale, kosmetische, Kompositionen ohne Etherphospholipide werden hier komponiert für sensible Personen, wobei die evidenzbasierten Problemen im Gebrauchsmuster offenbart sind. Schwangere, stillende Mütter mit Rücksicht auf Säuglinge, Kinder, Personen mit Haut, Darm, Leber, Gewichtsproblemen, Neigung zum Hochdruck, endothelialen Dysfunktion und/oder Glukoseintoleranz sollen keine alkoholartig transformierten Substanzen aufnehmen.

Die Zubereitungen von und mit Lecithinen ist auch geeignet zur schonenden Anreicherung von Fettsäuren, Omega-3-Fettsäuren, Flavonglycoside, Flavonoiden, Mevalonaten, Sterolen, Ginkgoloiden, energiereichen Spurenelementen, Phosphaten, wobei Antioxidantien immer vorhanden sind. Besonders gesunde Keimlinge werden zubereitete, die acetylierende Substanzen und Xanthine enthalten.

Alle bekannten Methoden für Testung, Zubereitung, Galeniken können genützt werden, auch um späte Transformationen zu vermeiden, indem kühl und abgedunketl angeboten wird. Vorzugsweise werden frische, natürliche Substanzen komponiert für milchige, cremige Kompositionen u.a. mit öligen Ginkgoloiden. Gereinigte Fisch- oder Pflanzenöle werden vorzugsweise mit zertifizierten Milchprodukten gemischt, die gesunde Acetylhydrolasen, Phospholipasen, Mineralien, Spurenelement und Vitamine enthalten. Die Kompositionen werden in nach außen einheitlicher Form angeboten mit einer originalen Marke und einem rosa Blatt (FiDA^{R}, EU-Marke No. 00108962).

Die vorliegende Patentanmeldung entwickelt entsprechende Zubereitungen und offenbart neue Herstellungsverfahren, die geeignet sind für sensible Personen, die keine weitere Aufnahme von Alkyl-Derivaten vertragen. Die Zubereitungen dienen vorzugsweise der reinigenden Pflege von Haut, Mund und Darm und/oder helfen Personen mit Alkohol-, Haut-, Gewichts-, Leber- und/oder Darmproblemen.

Die vorliegende Patentanmeldung offenbart die unerwünschte Wirkung von Alkoholmetaboliten und begegnet der Transformation von bekannten Mitteln. Keine unklaren Lipide, Phospholipide, Sterole werden eingeführt. Es ist neu und erfinderisch, Zubereitungen von Beginn auf ihren AAGPC-Gehalt zu prüfen. Es ist auch neu, alkylhaltige Vorstufen auszuschließen, die während der Zubereitungen Etherlipide, Alkylderivate bilden. Es ist besonders wichtig, die Grund- Zwischen- und Endsubstanzen zu testen, damit keine AAGPC-Derivate in die Zubereitung, Lagerung und Verwendung eingeführt werden. Die Testung der Grundsubstanzen und Hilfsstoffe ist besonders wichtig, weil eine nachträgliche Reinigung kaum möglich ist von den erwünschten Lecithinen (Acyl-2-acyl-GPC, phosphatidylcholine). Nur die Alkylgruppe in Position 1 von Glycerol unterscheidet die unerwünschten Alkyl-acyl-sn-glycero-3-phosphocholine (AAGPC) von den Lecithinen. Chromatografische Trennverfahren unterscheiden nicht ohne Weiteres. Enzymatische Zubereitung mit Phospholipasen können auch nicht unterscheiden zwischen Lecithinen und AAGPC-Phosphocholinen, die gleichermaßen gespalten werden durch Phospholipasen A1-A3,C,D. Der einzige Unterschied vom AAGPC besteht in den spezifischen Alkyl-Rezeptoren, so dass funktionelle Testungen nötig sind. Eine Nachbehandlung von verunreinigten Ölen ist bisher nur möglich mit löslichen Alkyl-Rezeptoren. Lösliche Rezeptoren und Antikörper zur Fällung sind bekannt (Y Fujimura et al. Athersoscerlosis 201, 108-11, 2008; AT Erkkilä et al. Atherosclerosis 2005, 183, 157-162). Die fixierten Zellen vom EP 06828720 legen Aufarbeitung der Alkylrezeptoren nahe.

Erstmals wird hier erfinderisch umgesetzt, dass Alkyl-Derivate, Etherlipide, Etherphospholipide gebildet werden durch fettigen Alkohol, weil ubiquitäre Enzyme in fast allen Zellen, Zellfragmenten, Bakterien, Hefen, Pilzen vorhanden sind. Wärmebehandlungen aktivieren diese ubiquitären Enzyme von Zellfragmente bei den üblichen wässrig-alkoholischen Verfahren, die hier erfindungsgemäß ersetzt wereden, indem kalt, kühl zubereitet wird.. Die Transformation von Inhaltsstoffen ist besonders unerwünscht für kosmetische, pflegende, dietätische Produkte sowie für ölige Produkte, Milchprodukte, Fischprodukte, Heilmittel.

Viele Verfahren benützten unklare Lösungsmittel, Emulgatoren, wogegen hier reine Lecithine genützt, angereichert und/oder verwendet werden. Geschmacksverstärker werden nicht eingeführt, die kritische Alkyl-Vorstufen in das Herstellungsverfahren einführen können. Biotechnologische Verfahren, die Zellen von Tieren, Pflanzen, Pilzen verarbeiten, sind besonders anfällig, weil Zellen, Zellinien, Bakterien Hefen ubiquitär Alkyl-Derivate bilden. Die gewünschten Beispiele für Kompositionen ohne Etherphospholipide, Alkyl-Derivate sind in den Ansprüchen vom Prioritätsdokument genannt, die hier zitierend einbezogen sind (vgl. Gebrauchsmuster Nr. 20 2011 108, Anm. 29.11.2011). Etherlipide werden hier erstmals ausgeschlossen.

Im vorpublizierten EP 06828720 werden neue Kompositionen ausgewählt mit neuen Methoden, weil erstmals Alkyl- und Acetylrezeptoren gerennt gestestet werden. Die Bildung von Alkyl-Acyl-GPC durch Lipide wird in vitro durch Serum Albumin gehemmt. EP 06828720 offenbart viele Methoden der Anmelderin, die zusammengestellt sind in einem Kapitel über die Hintergrundstechnik, die hier mit EP 0682870 zitierend einbezogen sind. Die Kompositionen vom EP 06828720 werden hier zitierend einbezogen und erfinderische Zubereitungen werden entwickelt.

Neu erkannt wird hier eine Verunreinigung mit Etherphospholipiden, die von Beginn bei/nach Zubereitung ausgeschlossen werden muss. Ausgangs, Endprodukte, Galeniken, Lagerung, unklare Hilfsstoffe werden überprüft und ersetzt um keine Alkyl-Gruppen in die Zubereitungen einzuführen.

Das Prioritätsdokument 20 2011 108 offenbart erstmals die exogene und endogene Bildung von Alkyl-Derivaten in der Anwesenheit von Zellen, Zellfragmenten, die mit fettigem Alkohol bei Wärme extrahiert werden und bezieht auch die analoge Metabolisierung von exogenen Alkyl-Derivaten ein. Es ist bekannt, dass die gewünschten Lecithine und Omega-3-Fettsäuren leicht transformiert werden bei der Herstellung, Lagerung und Verwendung. Transformierte Substanzen befinden sich häufig in Produkten, die mit Alkohol, Mikroben, Schimmelzellen, Hefen hergestellt werden oder in gerösteten Produkten. Alkyl-Derivate werden z.B. in Tier- und Pflanzenzellen gebildet, da die entsprechenden Enzyme stammesgeschichtlich alt sind. Herstellung, Lagerung, Einnahme der alten Mittel fördert die Bildung von Alkyl-Derivate. Wechselwirkungen, Emulgatoren oder Geschmacksverstärker können die Transformation verstärken. Nahrungsmittelzusätze erklären teilweise den epidemieartigen Anstieg von Gewichtsproblemen, dem hier zu begegnen ist. Lecithine mit Antioxidantien werden als handelsübliche, natürliche "Emulgatoren" verwendet, wobei nur synthetische Lecithine frei sein dürften von Etherphospholipiden (E304+E322). Andere Emulgatoren können dagegen Darmkrankheiten verschlechtern und Löslichkeitsverstärker sind meist nicht gekennzeichnet oder sind kritisch z.B. "Speisefettsäuren" (E470-477). Geschmacksverstärker sind für empfindliche Personen unerwünscht, da zum Beispiel Hefen neben dem Geschmacksverstärker Glutamat auch Alkyl-Derivate bilden können. Käse und besonders Schimmelkäse enthalten zu viele unklare Substanzen (vgl. J. Uribarri et al. J. Am. Diet Assoc. 2010; 110, 911-916). Das unerwartete Problem, wird in der vorliegenden Erfindung praktisch erkannt und erfinderisch gelöst.

Zum Beispiel entwickelten zwei ältere Frauen, die rauchten, nach bakteriellen Infekten einen Rheumaschub unter Einnahme vom handelsüblichen, klassischen Extrakt vom Ginkgo Biloba (40 mg/die, folgend EGB761). Die EGB761-Lösung wurde sodann verdünnt und diese schweren Störungen traten nicht mehr auf (1 Vol EGB761 (40mg) & 1 Vol. Öl ad 9 Vol. fettarme Milch).

Zwei jüngere Männer zeigten eine Verschlechterung der Akne vulgaris. Auf Wunsch der Patienten war dabei eine Rezeptur von dermalen Ginkgokompositionen angewendet worden, die in der Apotheke unter sterilen Bedingungen hergestellt wurde (1 Vol EGB761 ad 9 vol ph 5.5 Lotion). Die Rezepturen wurden dann geändert durch stärkere Verdünnung mit gereinigten antientzündlichen Lotionen (1ml GBE761 ad 200 ml Bepanthen-Lotion). Weitere Nebenwirkungen traten sodann nicht mehr auf bei den nächtlichen Anwendungen. Die dermalen, örtlichen Ginkgo-Kompositionen wurden ohne Lichtexposition angewendet, denn Pigmentstörungen , die im EP 06828720 offenbart sind.

Hier werden reine Grundsubstanzen von Beginn testend ausgewählt als wesentlicher Aspekt der Erfindung. Zum Beispiel werden hier zertifizierte Pflanzenöle (Walnussöl) ausgewählt, zubereitet und gemischt, die 72 % mehrfach ungesättigte Fettsäuren und davon 60% Omega-3-Fettsäuren enthalten (12% final). Gereinigte Keimöle aus ökologischem Anbau werden ebenfalls genützt (Keimöle, Nussöle, Leinöl, Sonnenblumen). Gesunde Keimlinge enthalten acetylierende Substanzen, Phosphate und/oder Ceramide. Weizenkeimlinge sind Energielieferanten und Sojakeimlinge liefern besonders langkettigen Ceramide. Diese Öle werden vorzugsweise mit Ziegenmilch und/oder Molkeprodukten gemischt, wobei das Volumenverhältnis zwischen 1 und 100 liegt insbesondere nahe 10 (1Vol Öl/ 9 Vol Milch).

Zertifizierte, pasteurisierte Milchprodukte werden vorzugsweise verwendet aus gesunder Tierzucht. Zum Beispiel werden fettarme Ziegen- oder Schafmilchprodukte verwendet, die Acetylhydrolasen enthalten und damit der Muttermilch ähnlich sind. Besonders gut eignet sich Ziegenmolke für die pflegende Reinigung bei kosmetischen oder gastroenterale Problemen. Besonders Ziegenmilch enthält gesunde Phospholipasen, Acetylhydrolasen, Lipasen. Die optimale Auswahl und Zusammensetzung der Komponenten wurde praktisch entwickelt und führt zu neuen, unerwarteten Kompositionen. Bei den neuen Zubereitungen werden die alkyl-spezifischen Enzyme und die enzymatischen, analogen Stoffwechselwege ganz praktisch durch Kälte, kühle Temperaturen gehemmt. Reine ölige Lecithine werden zubereitet, die sodann auch als Hilfsmittel verwendet werden z.B. für ölige Exraktion von gesunden Keimlingen oder von Blättern des Ginkgo-biloba. Organische Extraktionsmittel oder Emulgatoren werden weitgehend ersetzt. Immer wird mit Antioxidantien stabilisiert, wenn mit gereinigten Öle extrahiert wird, wobei eine ansteigende Kettenlänge eine Stufenextraktion erlaubt. Die ansteigend lipophilen Lecithine bestimmen die Polarität der gewünschten Pflanzenessenzen, die aufgenommen werden.

Eine weitere Neuheit besteht also in der stufenweisen, fraktionierten Extraktion von Lecithinen und in der schonenden Anreicherung z.B. von Flavonoiden und Fettsäuren. Ee bleibt sodann ein zellfreier, lecithinfreier, enzymfreier Zellrest, der sodann mit apolaren Lösungsmitteln mit den bekannten Methoden behandelt wird. Die Reinigungsverfahren werden vorzugsweise mit Enzymen, Proteinen Antikörpern durchgeführt. Diese apolaren Substazen z.B. Ginkgolide können dann ölig, cremig aufgenmmen werden vorzugsweise mit Pflanzenölen oder Pflanzenbutter. Für die enzymatische Ausreinigung ist sterile Ziegenmolke geeignet und sodann verunreinigtes Serum Albumin kann dn abgetrennt werden. Langkettige apolare Plasmalogenen, Ginkgolsäuren, Ginkgotoxine, Alkylphenole können enzymatisch gespalten werden auch mit synthetischen Phospholipasen, Esterasen. Phospholipase A1 spaltet die Acyl- und Alkylgruppen ab von Triglyceriden. Die unerwünschten Ginkolsäuren können mit Esterasen zerlegt werden. Die Spaltprodukte werden vorzugssweise mit steriler, fettfreier Albuminlösung oder mit steriler Ziegenmolke ausgewaschen und dafür sind alle Routinemethoden möglich (Zentrifugation, Ultrazentrifugation, Filtration, Ultrafiltration, Chormatografien, Säulen usw).

Die ölige Stufenextraktion fördert die Löslichkeit und erleichtert Galeniken zum Beispiel mit Dispersionen, Emulsionen, Liposomen, Vesikeln, Kapseln, Pasten, Cremes, Nanopartikel und auch Zubereitungen mit Nahrungsmitteln z.B. Milch, Kakao, Molke, Yoghurt, Buttermilch, Butter, alkoholfreie Getränke, Brei, Suppen.

Sehr wichtig sind Testungen vor und nach den Zubereitungen, Extraktionsstufen. Die neuen Verfahren dienen der Zubereitung von Zellen aus dem Tier-, Pflanzen-, Hefe-, Rekombinantenbereich und ersetzten übliche initiale Lösungsmittel z.B. Äthanol, Methanol, Buthanol, Hexane usw. Erstmals wird dabei beachtet, dass Zellfragmente mit ihren Mikrokörpern und Enzymen Alkyl-Derivate bilden in Anwesenheit von fettige Alkoholen schon bei Raumtemperatur.

Bislang blieb unbemerkt, dass Alkyl-Derivate auch in Heilmitteln gebildet werden können, die bei Raumtemperatur mit wässrig/ethanolischen Lösungen extrahiert werden. Im Hinblick die evidenzbasierten Risiken vom Prioritätsdokument muss nun der Nachweis verlangt werden, dass dietätische oder lokale Mittel für sensible Personen keine Alkyl-Derivate enthalten sollen. Fremde Verfahren werden folgend geprüft. Kritische fremde Verfahrensschritte sind zu ersetzten, besonders die wässrig-alkoholischen Extraktionsverfahren bei Wärme. Unnötige Trenn- und Reinigungsverfahren sind zu ersetzten durch Grundsubstanzen, die von Beginn aus ökologischer Pflanzen- und Tierzucht gewählt werden.

Die Zubereitung wird hier steril, kühl durchgeführt, weil besonders die Bakterien oder Pilze die unerwünschten Plasmalogene, Alkyl-Derivate in das Verfahren einführen. Herstellungs- und Lagerungsphase bei Umgebungstemperatur und/oder in Anwesenheit von kritischen Hilfsstoffen sind besonders kritisch, denn die Komponenten können auch nachträglich interagieren, transformieren und werden deshalb getrennt oder kühl und abgedunkelt gelagert. Es ist also wichtig, die Bildung von unerwünschten Substanzen in pflegenden, diätetische, lokalen und kosmetischen Zubereitungen zu hemmen oder zumindest stark zu vermindern, indem Hilfsstoffe vermieden werden, die oft Phosphocholine, Plasmalogene, Etherethanolamine enthalten. Der Nachweis muss erbracht werden für sensible Personen. Hier werden jedenfalls apolare Lösungsmittel, Wärmebehandungen, unklare Hilfmittel möglichst vermieden.

Die Neuheit der Erfindung besteht also im Wesentlichen in der initialen Auswahl von alkyl-freien Grundsubstanzen und sodann mit öligen Zubereitungen, die bei enzymhemmenden kalten, kühlen Temperaturen durchgeführt werden. Es wird im Kalten zerkleinert, gepresst und extrahiert. Die kühlen Extraktionsphasen verlangen einen längeren Zeitraum, in dem der Zellbrei z.B. gerührt, gequirlt, zerstampft wird. Das Verfahren dient vorzugsweise der schonenden Zubereitung von Lecithinölen, die auch ungesättigte Omega-3-Fettsäuren und/oder gesunde Phytosterole, und lichtabhängige Prähormone. Diese Substanzen sind empfindlich gegenüber Transformation mit Bildung von Alkyl-Derivaten, der Oxidation und der enzymatischen Betaoxidation. Für kosmetische Produkte ist die sterile schadstoffarme Zubereitung bekannt und besonders wichtig.

Die Präsenz oder Bildung von Alkyl-Derivaten stellt ein bislang unerkanntes Problem dar, das hier mit neuen, erfinderischen Zubereitungen und Kompositionen werden hier praktisch erkannt und erfinderisch gelöst für sensible Personen.

### Hintergrundstechnik:

Durch Lehrbücher ist es allgemein bekannt und nachvollziehbar, dass die enzymatische Bildung von Alkyl-Derivaten durch Einbau der Oxygene vom fettigen Alkohol in Acylglycerol vermittelt wird. Die Oxygene vom fettigen Alkohol ersetzten die veresterten Fettsäuren, die mit ihrem Sauerstoff freigesetzt werden und anfällig sind für Oxidationen. Die Enzyme zur Bildung der Alkylgruppen der Etherlipide, Etherphospholipide sind ubiquitär. Viele AAGPC-Plasmalogene werden gebildet z.B. durch Bakterien, Hefen, Tierzellen. Es ist zudem bekannt, dass Etherlipide, Etherphospholipide gebildet werden mit speziellen in vitro Verfahren zur semisynthetischen Aufarbeitung von Fischölen Rapsölen, Sojaölen usw (R Korth, HK Mangold, T Muramatsu & N Zöllner Chem Phys. Lipids 36, 209-214, 1985; SS Apte et al. & HK Mangold, FEBS Letters, 265, 104-106, 1990). Die Bildung von Alkylradikalen durch Pflanzen ist veröffentlicht (J. Shah, Annu Rev Phytopharm, 43,229-260, 2005). Besondere Vorsicht ist geboten bei Zubereitungen mit/von Bakterien, Zellinien oder Hefezellen, da diese Zellen, Zellfragmente Etherlipide und Alkylderivate bilden (H. Lux et al., Mol. Biochem. Parasitol. 1 11, 1-14, 2000). Die Alkyl-Phosphocholine werden bei der Schweinemast verwendet.

Bekanntlich bilden Zellmikrosomen Alkyl-Glycerole in der Gegenwart von langkettigen fettigen Alkoholen durch das Enzym Alkyldihydroxyacetonephosphate Synthetase (Alkyl-DHAP Synthetase) und Phosphotransferasen führen zu Alkyl-Derivaten (F. Snyder, Biological membranes (1988) Alan R. Liss Inc., pp 57-72). Die Bildung von Etherlipiden in menschlichen Zellen wird stimuliert mit Etherlipid-Mangelzellen vom Zellwegger Syndrom. Defiziente Fibroblasten werden mit dem peroxysomalen Enzym Alkyl-DHAP Synthetase(n) behandelt und Alkyl-Derivate werden über analoge Stoffwechselwege gebildet, so dass danach Etherphospholipide, Phosphocholine vorhanden sind (G.Schrakamp et al & H. van den Bosch, Journal of Lipid Research 26, 867-873, 1985). Das Optimum vom kritischen Enzym Alkyl-DHAP-synthetase liegt bei pH 8.5. Leider werden Etherlipide auch durch ubiquitäre Phosphotransferasen in Anwesenheit von 1-Alkyl-sn-Glycerolipiden gebildet (F. Snyder, Biological membranes (1988) Alan R. Liss Inc., pp 57-72). Diese Phosphotransferasen sind im alkalischen und sauren pH-Bereich aktiv (EF Bhungsgraberakd, E Chargraff, J. Biol. Chem. Vol. 242, N.20, 1967). Die pH-Verschiebungen von einzelnen Verfahrensschritten reichen daher nicht, so dass die kritischen Verfahrensschritte ins Kalte verlegt werden. Es wird kalt gemahlen, gepreßt und kühl extrahiert.

Das Prioritätsdokument beweist direkte Schäden durch die alkoholabhängigen Etherphospholipide. Klinisch ist die stabilisierende Einnahme von reinen Lecithinen bekannt für die alkoholbedingte Hepatitis (W. Lands, Alcohol Clin Exp Res, 1995). Die Lecithine und/oder mehrfach ungesättigten Fettsäuren werden aber endogen oxydiert und führen dann zum Abfall vom Schutzfaktor HDL, dem hier begegnet wird. Hier werden Lecithinöle schonend zubereitet und antioxidativ stabilisiert, denn Personen mit kritischem Alkoholkonsum bilden vermehrt Etherphospholipide und zudem oxidative Stressprodukte, die sodann gesunde Lecithine ersetzten in den HDL-Partikeln. Ein Ungleichgewicht entsteht im metabolischen System entsteht, dem hier mit den neuen Mitteln begegnet wird (RM Korth, Journal men's Health& Gender, 3; 279-289, 2006). Adipöse oder hyperlipidämische Patienten neigen zu Leberfunktionsstörungen, die durch stabile Alkoholmetabolite gesteigert werden. Die Gamma-GT von adipösen Männer mit Alkhoholkonsum steigt gefährlich an im Vergleich zu abstinenten, adipösen Männer (188±148 vs 65±3 ≥30 U/I). Auch hypertriglyceridämische Personen mit Alkoholkonsum vertragen keine Aufnahme von AAGPC-Phosphocholinen, die zu weiteren Leberschäden führen, was wird im Vergleich der alkoholbedingten zur nichtalkoholischen Hypertriglyceridämie hier gefunden wurde (127±129 vs 58±16 U/I Gamma-GT).

Ganz praktisch werden hier nur zertifizierte Speiseöle, Fischöle oder Lecithine verwendet mit definiertem Gehalt von Lecithinen, Omega-3-Fettsäuren, Antioxidantien. Geröstete Körner oder Schimmelpilze werden nicht verwendet, da viele unerwünschte Lipid Mediatoren vorhanden sind, die aufgelistet sind als transformierte Endprodukte d.h. "advanced glycation end products AGE" (vgl. D. Mozzafarian et al. Eur. J. Clin. Nutr. 201; 110, 911-916).

Die originalen Methoden schließen hier stabile Etherphospholipide testend aus, die sodann nicht in das Verfahren eingeführt werden, um Gewichtsproblemen zu begegnen, besonders der Adipositas. Das entsprechende US-Patent wird hierfür zitierend einbezogen (R. Korth, US Patent No. 5,895,785). Bekannte Kompositionen von spezifischen mit unspezifischen Paf-Antagonisten sind von von der Anmelderin offenbart. Eigenene frühe Kompositionen mit Ginkgoloiden, Ginkgoliden, Ginkgo Extrakten, Paf-Analogen und periphere Hetrazepine werden in der Patentanmeldung EP 06828720 als Hintergrundstechnik zusammengefaßt. Erstmals unterscheiden sich die öligen Kompositionen hier von der Hintergrundstechnik.

Die vorpublizierten Dokumente zeigen aber keinen Ausschluß von Etherphospholipiden bei der schonende Zubereitung von und mit Lecithinölen. Mit Lecithinölen werden viele Pflanzen- und Tierzellen stufenweise extrahiert, so dass apolare Lösungsmittel weitgehend unnötig werden. Nachweislich müssen Etherlipide, Alkyl-Derivate hier vermindert werden bei der Zubereitung von dietätischen, kosmetischen Kompositionen für sensible Personen. Tatsächlich beweisen die Alkoholschäden auch die Schäden der Alkyl-Verunreinigung, wobei stabile endogenen Alkoholmetaboliten zum signifikanten Gewichtsanstieg und zur endothelialen Dysfunktion führen (p<0.05). Diesen Problemen wird hier mit den erfinderischen Zubereitungen begegnet.

Das Hintergrundswissen wird in erfinderischer Weise entwickelt zu den neuen Zubereitungen, die entwickelt werden für noch gesunde, empfindliche Personen. Die Probleme und Behandlung sind im Prioritätsdokument 20 201 1 108 evidenzbasiert offenbart sind.

### Schutzrechte Dritter

Viele fremde Schutzrechte offenbaren Extraktionsmethoden und Extrakten aus Blättern vom Ginkgo biloba zur Behandlung der Demenz, aber der Wirkungsnachweis fehlt immer noch (B.Vellas et al. Lancet Neurology, 11, 851-859, 2012). Dagegen sind Schäden durch Alkyl-Derivate und die Transformation von Lecithinölen nachgewiesen, die nun auszuschließen sind. Besonders kritisch sind Ginkgo-Exraktionen, die auf wässrig-alkoholischen Extraktionen bei Raumtemperatur basieren (vgl EP 1 089 748, Anm. 19.6.1999). Alkyl-Derivate dürften gebildet werden, wenn bei Raumtemperatur mit wässrig-alkoholischen Lösungen extrahiert wird ohne Rücksicht auf aktive Enzyme und/oder Zellfragmente, die bei Wärme in wässrig-alkoholischen Lösungen Alkyl-Derivate bilden. Klassische Ginkgo-Extrakte, die mit unklaren Phospholipiden hergestellt werde, verursachen Kopfschmerzen oder können zu Epilepsien führen (vgl. EP 0 275 005, Anm. 20.7.1988). Dagegen nützt die vorliegende Erfindung Kälte zur Enzymhemmung. Es wird kalt gemahlen, gepreßt, zubereitet und gelagert. Andere frühe Schutzrechte extrahieren lipophiles Bilobalid und benützen Emulgatoren z.B. Cetylstearylalcohol (DE 3338995, Anm. 9.5.85). Fortlaufende Patente basieren dann auf Acetonextrakten und führen unklare Lösungsmittel ein (EP 0431 536, Anm. 3.12.1990; DE 39 40 091-09, Anm. 4.12.89). Diese fremden Patente konzentrieren sich auf die Ausreinigung von Ginkgolsäure, Ginkgotoxinen (Samen) und von Alkylphenol-Pestiziden. Zumindest sollten die Pestizide durch Auswahl von ökologischen Ginkgo-Kulturen von Beginn ausgeschlossen sein.

Andere noch valide Schutzrechte mischen Ginkgo-Hexanextrakte mit Polyphenolen der Kaffeesäuren oder von Alep-Nüssen um den Schäden der Haut zu begegnen, die durch freie Radikale verursacht werden (vgl. EP 0627 909). Kaffee und Nüsse neigen zur Schimmelbildung und unklare Hilfsmittel werden eingeführt. Die Extrakte bei Wärme sind also anfällig, aber nur die Autoxidation von Fettsäuren (Vitamin F) wird gemessen. Hier müssen Etherphospholipide nachweislich ausgeschlossen werden. Bakterielle, fungale Verunreinigung sind besonders auszuschließen bei nussigen Grundsubstanzen. Grüner Tee kann Pestizide enthalten und der nachweisliche Ausschluß fehlt. Die neuen Herstellungsverfahren nützen schonende Verfahren für lokale und dietätische Kompositionen mit schrittwiser öliger Extraktion. Die Kompositionen der Anmelderin wurden, werden mit den validen Marken der Anmelderin gekennzeichnet, wobei die Fida-Marken wieder ausdrücklich und ungeteilt in Anspruch genommen werden (R. Korth, valide EU-Marke No. 00108962 und Fida^{R}, Fidaderm^{R}, Fidabus^{R}).

Die vorliegende Erfindung extrahiert mit Ölen, die Lecithine, Omega-3-Fettsäuren und Antioxidantien enthalten (Tocopherole). Mit diesen Ölen werden dann andere Öle fraktioniert hergestellt, wobei Lecihtinöle auch als Extraktions- und Lösungsmittel verwendet werden, z.B. um den Löslichkeitsproblemen von Ginkgoliden zu begegnen.

### Herstellungsverfahren

Gegenstand der Erfindung ist die Hemmung von neugebildeten Alkyl-Derivaten bei der Zubereitung von pflegenden, dietätischen, kosmetischen Produkten, die vozugweise Lecithine, Omega-3-Fettsäuren, Vitamine, Mineralien, Spurnelemente, energiereiche Substanzen mit Xanthinen und die mit antioxidativem Serumalbumin gemischt werden sollen. Milchprodukte fügen gesunde Inhaltsstoffe hinzu für sensiblen Personen. Die erfinderischen, neuen Zubereitungen werden zunächst mit verdünnenden Rezepturen zubereitet, indem zertifizierte Pflanzenöle ausgewählt werden aus ökologischen Anbau und pasteurisierte, zertifizierte Milchprodukten aus gesunder Tierzucht, die unter sterilen Bedingungen gemischt werden.

Gute Löslichkeit wird hier von Beginn erreicht durch Lecithinöle, die auch als reine synthetische Öle im Handel angeboten werden mit Antioxidantien und die dann ölige Extrakte aus Pflanzen fördern. Ölige Galeniken werden besser resorbiert von der Haut oder vom Darm. Die vorliegende Erfindung exrahiert vorzugsweise mit reinen pflanzlichen Lecithinölen, die nachweislich keine Etherphospholipide enthalten.

Die öligen Extraktionsschritte ersetzten fremde Herstellungsverfahren ganz oder teilweise. Die gewünschten Lecithine (Phosphatidylcholine) werden hier schonend benützt und auch extrahiert für Kompositionen mit Lecithinen, Flavonoiden, fettlöslichen Vitaminen, die mit Milch-, Molkeprodukten ergänzt werden zur Anreicherung von Mineralien, Sterole, Enzymen. Acetylhydrolasen, Phospholipasen spalten auch endogene Etherlipide, Etherphospholipide möglichst schon im Mund, Magen, Darm und auf der Haut. Die Kompositionen schützen Personen mit Gewichts-, Haut- und Leberproblemen. Unklare industrielle Hilfstoffe, Löslichkeitsverbesserer, Hilfsmittel werden vermieden oder ersetzt durch zertifizierte natürliche Substanzen, um der epdidemieartigen Gewichtsproblemn zu begegnen.

Die initiale Auswahl von reinen Ölen und Milchprodukten ist entscheidend. Zellen der Tier- und Fischwelt werden verarbeitet unter der Bedingung von gesunden Kultur- und Zuchtbedingungen, so dass viele Reinigungsschritte unnötig werden. Zellen, bzw. Zellprodukte aus dem Pflanzen-, Tier, Hefebereich werden initial im Kalten zerkleinert, ausgepreßt, so dass reine Lecithinöle stufenweise extrahiert werden, die dann auch weitenrerändert werden. Die erfinderischen Schritte können gleichzeitig, nacheinander und in zweckmäßiger Reihenfolge durchgeführt werden und dienen der verbesserten Herstellung, Lagerung, galenischen Bearbeitung. Verwendung von Ölen und Milchprodukten werden als Kompositionen für Personen zubereitet, die keine Alkyl-Derivate mehr vertragen (z.B. bei Haut-, Gewichts-, Leber- und Darmproblemen).

Folgende Schritte kennzeichnen die Erfindung zur Aufarbeitung von Pflanzen- oder Tierzellen. Man beginnt mit reinen, zertifizierten Substanzen ohne Lösungsmittel, Geschmacksverstärker, Emulgatoren, transformierte, ranzige, oxidierte Bestandteilen. Die Etherphospholipide werden ausgeschlossen mit den anerkannten Methoden der Anmelderin. Im Vordergrund steht die enzymatische Hemmung durch möglichst kühle Temperaturen beim Zerkleinern und Zubereiten. Die laufende Testung erspart unnötige Verfahrensschritte.

### Verfahren I

Die Auswahl der Grundsubstanzen verlangt reine, ökologische Naturstoffe ohne Zusatz von Hefe, Bakterien Emulgatoren. Alkyl-Derivate werden gestestet. Das nachgenannte Verfahren bietet die besten Resultate für Pflanzenöle. Die Antoxidantien sind in allen Verfahrensschritten vorhanden (Ascorbinsäure, Tocopherole usw)..
a) Die ethanolische Extraktionsverfahren werden vorzugsweise ersetzt durch physikalische Methoden, die bei möglichst kühlen Temperaturen durchgeführt werden (z.B. Ultraschall, Pressen, Mahlen).
b) Sodann wird der Zellsaft vom Zellbrei getrennt. Vorzugsweise durch Zentrifugation oder Ultrazentrifugation werden Zellbestandteile abgetrennt vom Primärextrakt, der kalt und dunkel gelagert wird (Extrakt/Öl 1). Dieser Primär-Extrakte enthält hydrophile und ölige Substanzen der Zellen d.h. Fettsäuren, Flavonoide.
c) Apolare Lecithine bleiben eher membrangebunden. Die sedimentierten Zellfragmente werden enzymhemmend kühl verarbeit. Die Alkyl-DHAP Synthetasen werden gehemmt durch saure pH-Werte und die Phosphotransferasen durch Kälte (z.B. 4 ° C, pH 6.5). Die kalte Zellsuspension wird vorzugsweise mit reinen, kühlen C-16-Lecithinölen aufgenommen, wobei die kühle Extraktionsphase effizienter wird durch Rühren, Quirlen, Pressen, Zerstampfen über einen verlängerten Zeitraum (z.B. >24 h, bei 4 ° C ). Das zugeführte Volumen vom synthetischen C-16 Lecithinöl ist möglichst niedrig, um Zellsaft bzw. die Zellöle nicht zu verdünnen. Wünschenswert wäre, dass zum Beispiel Keimlinge mehrmals kühl gepreßt und gelöst werden, um zelluläres C-16-Lecithin-Öl zu gewinnen. Sodann werden die Zellfragmente vorzugsweise zentrifugiert oder ultrazentrifugiert und der native Überstand (Öl II) wird kalt gelagert, der nun natürliche C16-Lecithine enthält.
d) Die sedimentierten Zellfragmente werden mit langkettigen Lecithinen (C18-) aufgenommen, wobei die Polarität vom Öl -Fett der Polarität vom gewünschten Zellbestandteil entspricht. Dieser Verfahrensschritt wird bei ansteigender Wärme durchgeführt zur besseren Fettlöslichkeit und auch weil kritische Enzyme nun z.B. durch Ultrazentrifugation abgetrennt wurden. Wünschenswert ist z.B. eine Extrakton mit und von C18-Lecithinölen bei etwas erhöhter Raumtemperatur, um die langkettigen Lecithine oder Ceramide zu extrahieren. Temperatur und Zeitraum wird angepaßt (z.B. 30 Min). Danach wird der ölige Zellbrei wieder abgekühlt und mehrmals gepreßt und gelöst, um sodann möglichst mit kühler Zentrifugation oder Ultrazentrifugation das Öl II abzutrennen, das nun vorzugsweise C-18 Lecithine und langkettige Ceramide enthält. Das wertvolle Öl II wird kühl und dunkel gelagert.
e) Der restliche sedimentierte Zellrest enthält apolare Substanzen, die sodann mit organischen Lösungsmitteln bei Wärme behandelt werden können, weil keine schützenswerten Lecithine, Fettsäuren, Ceramide, Flavonoide und auch keine aktiven Enzyme mehr vorhanden sind. Die organischen Mittel z.B.Aceton-Wasser werden evaporiert, die Zellsuspensionen wird z.B. mit C-18-Öl aufgenommen und sodann wird enzymatisch gereinigt z.B. mit steriler Ziegemolke oder mit synthetischen Substanzen, die Phospholipasen A1,A2,C,D enthalten. Lipasen degradieren Neutralfette und Esterasen störende Phenol-Derivate, die dann mit Routinemethoden entfernt werden. Es entsteht ein apolarer Extrakt, der als apolares Öl/Fett III kühl, dunktel gelagert wird.

Diese Zubereitung nützt Kälte und Kühle für kritische Verfahrenschritte, auch für Zentrifugieren oder Ultrazentrifugation und erspart damit Gradientenmittel. Mögliche Etherlipide, Etherphospholipide werden getestet Die reinen nativen Öl/Extrakten I-III werden gemischt oder getrennt gereinigt, verarbeitet. Die Öle I-III können z.B. auch genützt werden für Extrakte von anderen Pflanzen zum Beispiel von gesunden Keimlingen und/oder von Blättern des Ginkgo biloba. Auch bei der Lagerung oder der Galenik können noch Etherlipide, Akyl-Derivate entstehen, so dass die Kühlkette nicht unterbrochen wird oder sonst Kombinationspackungen die Interaktion, Transformation vermindern. Milch/Molkeprodukte mit Phospholipasen, Acetylhydrolasen sind von lecithinhltigen Ölen zu trennen getrennt zum Beispiel durch Kombipackungen. Die Zentrifugation oder Ultrazentrifugation wird hier bevorzugt, weil sie kühl durchgeführt werden kann ohne Verdünnungen.

Eine nachträgliche Ausreinigung mit löslichen Alkylrezeptoren ist zwar möglich aber noch kein Standardverfahren. Antikörperpräzipitationen sind als bekannte Reinigunsverfahren möglich (Säulen usw). Die erfinderischen Verfahrensschritte ergänzen, ersetzeneinzeln sodann bekannten Extraktionsverfahren.

### Verfahren 2:

Das nachgenannte sterilen Verfahren bieten die besten Resultate für kosmetische, pflegende Kompositionen. Ausgangssubstanzen mit Risiko von Schimmel, Pestiziden usw werden ebenfalls von Beginn ausgeschlossen.
f) Nur reine, zertifizierte Öle und Trägersubstanzen werden ausgewählt, die keine Etherphospholipide, Alkyllipide enthalten.
g) Das o.g. Verfahren gewinnt gereinigte Lecithinöl I-III, die dann fraktioniert verwendet werden für weitere Extraktionen, wobei die langkettigen Ceramide und energiereichen Substanzen gewünscht sind, die Actyl CoA und Xanthine enthalten (z.B 1 Vol Sojakeimlinge und 10 Vol. Kakaobutter).
h) Für die galenischen Aufbereitungen werden die ansteigend fettigen Öle gemischt oder getrennt verarbeitet.
i) Ziegenmolke oder equivalente Substanzen werden für reinigende Pflege verwendet und/oder für die lokale enzymatische Reinigung (z.B. 1 Vol. Sojakeimöl und 100 Vol Ziegenmolke-).
j) Fetthaltige Milchprodukte oder Pflanzenbutter wird ebenfalls verwendet und vorzugsweise mit Pflanzenöl III gemischt.

### Verfahren 3.

Die o.g. Verfahren können weiter genützt werden zur öligen Extraktion von Ginkgo-Bestandteilen, wobei z.B. reine Lecithinöle stufenweise und kühl verwendet werden.
a) Die Ginkgo-Bestandteile aus ökologischem Anbau werden mit Ultraschall im Kalten zerkleinert und dann kalt gepreßt. Ethanolische Extraktionsmethoden werden ersetzt und falls nötig nur bei Kälte durchgeführt mit Rotation und verlängerter Inkubation (z.B. bei 4° C, >24 h). Es entsteht der ausgepreßte Zellextrakt I aus Blättern. Bei Samenextrakten muss das Zellöl I besonders untersucht und ausgereinigt werden.
b) Zu Beginn werden Blätter vom Ginkgo billoba kühl und möglichst mechanisch zerkleinert. Sodann wird der Zellbrei mit C16-Lecithinölen über längere Zeit kühl, gerührt, gequirlt, gestampft, ausgepreßt, gelöst (>24 Std bei 4° C). Das Primäröl I nimmt antioxidative Flavonoide und Lecithine auf, die im Überstand bleiben und kühl gelagert werden. Vorzugsweise die Zentrifugation oder Ultrazentrifugation bei Kälte trennt die kritischen Zellmikrosomen und Enzyme ab, so dass der nächste Schrittt bei Wärme durchgeführt werden kann.
c) Der sedimentierte Pflanzenbrei wird mit C18-Lecithinöl aufgenommen und bei Wärme inkubiert, um sodann gemäß Schritt b abzentrifugiert zu werden. Das apolarere Öl II enthält das antioxidative Bilobalid und wird im Kalten dunkel gelagert.
d) Der restliche Pflanzenbrei III enthält weder Lecithine noch Enzyme und kann mit den bekannten apolare Extraktionsmethoden durchgeführt werden auch bei Wärme zur Anreicherung von Ginkgoliden. Hexanbehandlung oder Wasser-Aceton mit folgender Evaporation sind erpobt und möglich.
e) Eine enzymtische Reinigung der Phase III bevorzugt hier Lipasen, Phospholipasen und Esterasen, um verbleibende AAGPC-Etherbindung zu spalten, die auch in Neutralfetten vorkommen. Esterasen spalten Esterbindung der Ginkgolsäuren, Ginkgotoxine Alkylphenole. Die unerwünschten Bruchstücke werden mit fettfreier, steriler Albuminlösung vorzugsweise mit steriler Ziegenmolke ausgewaschen. Die unreine Albuminlösung kann problemlso abgetrennt werden von den apolaren Substanzen z.B. durch kühle Zentrifugation oder Ultracentrifugation.
f) Die gereinigten Ginkgolide werden mit einem apolaren Fett bei Wärme aufgenommen (z.B. mit sterile Pflanzenbutter)

Alle Schritte können angepaßt, ergänzt werden und ersetzen einzeln und getrennt die bewährten, freien Verfahren. Als Trennungsverfahren können Sedimentation, Filtration, Ultrafiltration, Dialysen, Säulentrennung, Antikörperbehandlung, Chormatographien und bekannte Routineverfahren genützt werden.

Die öligen Extrakte I-III können getrennt oder gemeinsam geprüft, gereinigt, angereichert werden. Vorzugsweise werden gesunde Lecithinöle der Faktion III mit mit Ginkgo-Ölen der Fraktion I-III gemischt im besonderen Hinblick auf die Löslichkeitsproblme der lipophilen Ginkgolide. In dieser Weise entstehen Micellen, so dass sich Lösungmittel erübrigen.

### Beispiele Pflanzenölen und Milchprodukte:

Die Beispiele 1-3 komponieren beispielhaft zertifiziertes Walnußöl und Ziegenmilch. Alle zertifizierten Pflanzenöle vom ökologischen Anbau können benützt werden und alle Milchprodukte, die aus gesunder Tierzucht (bis auf Käse). Equivalent Substanzen sind möglich. Die folgende Beispiele bereiten ein gesundes Verhältnis zu von Serum-Albumin, Lecithin, Vitaminen, Omega-3-Fettsäuren und Antioxidantien und können mit gewünschten Substanzen angereichert werden, die aus gesunden Keimlingen, Kakao und/oder Ginkgoblättern zubereitet werden.

### Beispiel1:

| | |
|---|---|
| 2 g | Öl I+II aus gereinigten Walnußölen |
| 8 g | Öl III |
| 100 g | Ziegenmilchjoghurt |

### Belspiel2:

| | |
|---|---|
| 2 g | Öl I+II aus gereinigten Walnußölen |
| 8 g | Öl III |
| 100 g | fettarme Schafmilchjoghurt |

### Beispiel 3 zur Mund-, Darmreinigung, auch bei Glutenunverträglichkeit:

| | |
|---|---|
| 2g | Öl I+II aus gereinigten Keimlingen (z.B. Sojakeimlingen) |
| 2 g | Öl III |
| 100 g | fettarme Ziegenmolke |

Besonders zertifzierte Ziegen- und Schafmilchprodukte werden gewählt, um Enzyme zu nützen, wobei besonders energiereiche Keimlinge oder Pflanzenöle mit Omega-3-Fettsäuren gewählt werden. Diese Zubereitung kann den Alkoholkonsum neutralisieren, den Gewichtsanstieg mildern, der ansonsten signifikant ist (p<0.05). Die Kompositionen enthalten natives Serumalbumin, Phospholipasen, stabile Lecithine, Omega-3-Fettsäuren. Alkoholmetabolite werden os zumindest teilweise im Darm abgebaut und/oder über den Urin ausgeschieden. Milch enthält Prähormone wie z.B. Dehydrocholesterole, Mineralien, viel Calcium, Phosphor Magnesium und viele Vitamine (Vit. C-F). Milch kann mit Kakao gemischt werden für Kinder. Gesunde Yoghurtkulturen werden auswärts kontrolliert. Die erfindungsgemäßen Kompositionen mit Serum Albumin stabilisieren gesunde Lecithine zum Wohle vom Endothel, Darm, Haut, Zellen, Gewebe, Leber, HDL-Lipoproteinen, Serum Albumin. Erfindungsgemäß werden hier die Etherlipide, Ether Phospholipide, Alkylradikale im Darm neutralisiert mit Milch- und Pflanzenprodukten, ohne Alkylradikale oder Entzündungsmediatoren zu bilden. Gesunde Komponenten wie Folsäsure, Jodid können angereichert, ergänzt, zugefügt werden. Besonders ältere Frauen brauchen zudem Ginkgoloide, Flavonglycoside, Prähormone, Phytohormone, Jodid, Mineralien, Vitamine, die angereichert, ergänzt, zugefügt werden. Besonders das apolare Öl III kann Ginkgolide extrahieren und zumindest als Micellen lösen oder mit Pflanzenbutter (Kakaobutter) verabreiht werden. Die Ölfraktionen I und II eignen sch für Lecithine, Ceramide und Fettsäuren.

**Beispiele Schönheits-, Pflegemittel und Kosmetika mit den gereinigten Ölen:**

### Beispiel 5 zur Pflege

| | |
|---|---|
| 2 ml | Walnußöl (Öl I+II) |
| 2 ml | Öl III |
| 100 ml | stabiliserte Körpermilch mit Vitaminen und Antioxidantien |

| | |
|---|---|
| (z.B. Bepanthen, Bayer, Deutschland) | |

### Beispiel 6 zur Pflege

| | |
|---|---|
| 2 ml | Sojaöl (Öl I+II) |
| 2 ml | Sojaöl III |
| 100 ml | stabiliserte Creme mit Vitaminen und Antioxidantien |

### Beispiel 7: Rezeptur zur reinigenden Pflege der Haut.

| | |
|---|---|
| 1 ml | Öl I+II |
| 2 ml | Öl III |
| 100 ml | Ziegenmilch oder Ziegenmolke |

Erfindungsgemäß werden Öle gewählt oder zubereitet, die keine Alkyl-Derivate enthalten. Gewünscht sind Ceramide, Fettsäuren, native Lecithine , die schonend zubereitet werden.

Ginkgolide und Flavonglycoside können dem apolaren Öl III zugefügt werden, um sodann mit einer Pflanzenbutter eine Creme oder mit Pflanzenölen eine milchige Komposition zu bilden. Das Öl III kann Ginkgolide extrahieren, so dass auch die kosmetischen Beispiele 6 und 7 mit öligen Ginkgo Extrakten, Ginkgoloiden angereichert ohne schädliche Lösungsmittel.

Serum Albumin, Antioxidantien und Vitamine ohne transformierte Substanzen werden mit Pflanzenölen z.B. energiereichen Keimölen, Nussölen, fettigen Palmölen, ceramidhaltigen Sojaölen oder Leindotterölen, Kürbiskernölen, Sonnenblumenölen, Hanfölen, Leinölen, gereinigtem Olivenöl und/oder mit synthetischen Ölen zubereite. De Öle enthalten ungesättigte Fettsäuren, Lecithine und Antioxidantien und Tocopherole und gewünschte Komponente können zugefügt werden.

Besonders Personen mit Neurodermitis, Akne vulgaris, intertriginösen Problemen d.h. Personen mit bakteriellen oder fungalen Hautproblemen lokalen Kompositionen werden behandelt. Zudem können z.B. beruhigende Komponenten auch gekühlt angewendet werden. Produkte der Ziegenmolke sind zur enzymatischen Hautreinigung geeignet von sensiblen Personen, denn Acetylhydrolasen bauen Etherphospholipide ab auch im sauren pH-Bereich. Die pflegenden, kosmetischen Kompositionen, die durch den Ausschluss von Etherlipiden, Etherphospholipiden, Alkylradikalen gekennzeichnet sind, können als Kombinationspackungen angeboten werden in nach außen einheitlicher Form. Das Warenzeichen Fida schützt die Anmelderin vor Kopien und Nachpatentierereien, wobei die FiDA-Marken wieder ausdrücklich und ungeteilt in Anspruch genommen werden (*FiDA*^{R}).

### Beispiel 8:

### Angereicherte Fischöle:

Fettarme Milch (1l; 1.5%, 1300 mg Calcium u. 1000 mg Phosphorus, Vitamin C 1700 mg/100 ml, Prähormonen) mit Zugabe von 0.8 mg Folsäure/Tag Zertifizierte Fischöle equivalent zu 100 g Hering pro Tag, mit zumindest 10 Gew% Omega-3-Fettsäuren, 30*µ*g Cholecalciferol und 100*µ*g Jodid.

Nur zertifzierte Fischöle ohne Schadstoffe werden hier angeboten, bei denen Pestizide, Schadstoffe, Harnsäure, negative Spurenelement von Beginn ausgeschlossen sind. Die Fischprodukte werden durch Milchprodukte ergänzt, denen Folsäure zugesetzt wird. Die gereinigten Fischöle werden angereichert mit Jodid und Cholecalciferolen. Fisch vorzugsweise Seefisch liefert Prähormone wie Dehydrocholesterole und möglichst auch Jodid. Dagegen enthalten Fischöle nur ungesättigte Fettsäuren ohne weitere gesunde Substanzen. Reine Fischöle mit Omega-3-Fettsäuren benötigen auch die Zugabe von Antioxidantien.

Schwangere Frauen brauchen zusätzlich Vitamine wie Folsäure, Calcium und Jodid. Zertifizierte Fischöle werden also angereichert, ergänzt werden, wobei die Kombination von zertifizierten Fisch- und Milchprodukten besonders wichtig ist für schwangere Frauen. Möglichst natürliche Dehydrocholesterole sind einzunehmen bei erhöhter Calciurese und/oder Albuminurie. Alkoholmetabolite sind embryotoxisch, so dass Etherphospholipide auszuschließen sind, die zudem die Wehentätigkeit beeinflussen.

### Beispiel 8. Ginkgoloidöle

Durch ansteigend apolare reine Öle werden apolare Ginkgolide gelöst z.B. als Micellen oder mit Pflanzenbutter (z.B. 1 Vol Ginkgolodeöl und 10 Vol Kakaobutter). Die Ginkgoloidöle können der apolaren Phase III der vorgenannten Beispiele aufgenommen werden und bilden sodann milchig, cremige Zubereitungen.

### Beispiel 9:

| | |
|---|---|
| 3g | Gingkolid Öl I (Flavonoide)) |
| 2 g | Ginkgoloid Öl II (Bilobalide) |
| 1 g | GinkgoloideÖl/Fett III (Ginkgolide) |

Die Ginkgolidöle I-III werden gemischt und dann mit den Ölen III der Beispiele 1 bis 7 gemischt, so dass sich lösliche Ginkgoloid-Micellen bilden odr Ginkgoloid-Creme.

### Beispielgruppe 10:

Öle der Gruppe III aus den Beispielen 1-7 werden genützt zur Extraktion der aus Ginkgoblättern

| | |
|---|---|
| 100 g | Öle aus den Beispielen 1-7 werden danach gemischt mit |
| 1 g | Ginkgoloidöl (I-III) |

Es entstheht mit Pflanzenbutter eine Creme und sodann über Micellen eine Milch, die dann mit den o.g. Ölen, Milchprodukten gemischt werden kann.

Die Reinheit von Beginn ist entscheidend von Beginn bei der Auswahl der Ginkgobaumschulen. Auch warme Lagerung und/oder lange Transportwege aus China und Korea sind zu vermeiden. Vorzugsweise alkyl-freie Blätter werden hier ausgewählt, kalt zerkleinert gepreßt und mit ansteigend apolaren Mitteln extrahiert über eine längerne Zeitraum. Eine Wasser/Acetonextraktion wird nicht am Anfang sondern am Ende durchgeführt. Die enzymatisch inaktive Restsubstanzen werden vorzgusweise mit Phospholipasen und Esterasen gereinigt (vgl. T. Hamers et al., Environ Pollut 123, 47-65, 2003). Sterile entfettete Ziegenmolke stellt ein kostengünstige Reinigungsmittel dar, das endogen und exogen die Phospholipide degradiert. Sodann sind die verunreinigenden Spaltprodukte leicht abzutrennen.

Synthetische Lecithinöle oder Rekombinantenprodukte z.B. Rekombinantalbumin können bei Zubereitung hilfreich sein, wenn die Rekombinantenmittel gründlich entfernt werden.

Besonders kosmetische Mittel, sowie dietätische Mittel für stillende Mütter und deren Säuglinge und auch Kindernahrung müssen hohen Anforderungen genügen. Entsprechende Verträglichkeitsstudien und Zertifikate sind erwünscht und nötig.

### Klinik und Diskussion:

Empfindliche Personen vertragen die Aufnahme von Etherphospholipiden nicht. Personen mit kritischen Lipidprofilen, kritischen Glukoseprofilen, Neigung zum Hochdruck, Nikotin- oder Alkoholkonsum, Pigmentstörungen sowie alternde Personen, Personen mit Stuhlunregelmäßigkeiten, Darmerkrankungen, Hautproblemen sind empfindlich. Diese Personen sind besonders empfindlich, wobei eine erhöhte Empfindlichkeit mit kritischen Morgenurinen, der endogene Bildung von Ether-phospholipiden erkannt werden. Auch Testungen von Blutkompartimenten, Stuhluntersuchungen, Gewebebiopsien sind sinnvoll (Leber, Fettgewebe). Eine Zusammenfassung der bekannten Methoden, Kompositionen und Verwendungen der Anmelderin sind mit der Hintergrundstechnik in der Patentanmeldung EP 06828720 offenbart.

Nebenwirkungen vom EGB761 wurden beobachtet bei sehr empfindlichen Personen mit endogener Bildung von Etherphospholipiden, denn ander Lpidmediatoren sind nicht so stabil und nicht so virulent. Die erfindungsgemäßen Zubereitungen verdünnen für sensible Personen, die bereits unter der endogenen Produktion von Alkyl-Derivate leiden, so dass eine vermehrte Aufnahme von transfomierten Substanzen besonders ungeeignet ist. Die örtlichen und dietätischen Kompositionen begegnen vorzugsweise endothelialen Störungen durch Alkohol, falsche Ernährung, Rauchen und metabolische Symptomen von sensiblen und/oder älteren Personen. Die Kompositionen begegnen besonders den frühen endothelialen Schäden durch Aufnahme oder Bildung von stabilen Alkoholmetaboliten und/oder von oxidierten Substanzen. Die transformierten, alkylierten Substanzen sind zu vermindern besonders der Phospholipide, die endogen und exogen gebildet werden. Etherphospholipide ähneln den schädigenden Alkoholmetaboliten, die endotheliale Störungen, Gewichtszunahme, Absenken vom HDLvermitteln und die Leberbelasten. Die Mittel gleichen auch Leberstörungen aus und stärken den endogenen Schutz.

Erfindungsgemäß werden lokale, kosmetische, pflegende, dietätische Zubereitungen mit gesunden Lecithinen, Fettsäuren, Serum Albumin hier schonend angereichert ohne Alkylglycerole, Alkyl-Derivate, Etherlipide, Etherphospholipide. Von Beginn werden reine Grundsubstanzen ausgewählt, die sodann im Kalten zubereitet werden, damit nicht alkylartig transformiert wird. Nur mit alkylfreien Hilfsmitteln wird im Kalten die Inkubation, vorzugsweise eine Langzeitinkubation durchgeführt mit ansteigend langkettig veresterten reinen Lecithinölen, die sodann mit Grundsubstanzen aus Milch, Fisch, Pflanzen mit deren Zellen, Zellbestandteilen komponiert werden. Die Zubereitungen nützen zelluläre Bestandteile und bilden keine Etherlipide, Etherphospholipide, Alkylderivate.

Von Beginn werden schädigende Substanzen ausgeschlossen, so dass keine Alkylphenol-Pestizide, Schimmel, Bakterien, Entzündungsmediatoren (Endotoxine) eingeführt werden. Es werden keine geröstete, geräucherten Produkte verwendet, keine Schimmelkässe. Nur frische Milch-, Fisch-, Pfanzenprodukte werden zubereitet, die gesunde ungesättigte Fettsäuren, Lecithine, Antioxidantien, Flavonglycoside, Prähormone, Phytohormone, Hormone enthalten, die angereichert, zugefügt, unverändert zubereitet wreden, wobei equivalente Substanzen einbezogen sind. Ginkgoloide, Mineralien, Vitamine, Jodid, energiereische Substanzen (Acetyl CoA und Xanthine) werden nach Bedarf den o.g. Zubereitungen zugegeben. Dietätische Kompositionen werden mit schadstoffarmer Nahrung angeboten, die einen niedrigen Gehalt von schädigenden Fetten, Salzen, Carbohydraten haben und ohne Alkohol. Alkoholmetabolite bilden endogen Etherlipide, Ether Phospholipide, Alkyl-Derivate schon im Darm und mit fast jedem Nahrungsmittel, so dass Milchprodukte mit Phospholipasen, Acetylhydrolasen schon im Mund im Darm die schädigenen Etherlipide, Phospholipide abbauen sollen. Die gewünschten Komponenten sind Serum Albumin, ungesättigte Fettsäuren, Lecithine, Antioxidantien, Vitamine, Folsäure, Mineralien wie Calcium, Jodid und Prähormonen, Dehydrocholesterolen, die auch in Milch/Molkeprodukten vorhanden sind. Besonders Ziegenmolke wird verwendet. Gewünschte Produkte werden gewählt, angereichert, zugefügt aus der Gruppe vom Dehydrocholesterol, der Antioxidantien, der Ginkgoloide, der Flavonglycoside, der Vitamine, Mineralien, Enzyme enthalten und auch gesunde Lipasen, Phospholipasen, Acetylhydrolasen zur reinigenden Pflege von Mund, Darm und Haut. Zertifizierte Pflanzenöle werden gewählt wie Kernöle, Rapsöle, Palmöle, Sojaöle, Leindotteröle, Kürbiskernöle, Sonnenblumenöle, Hanföle, Leinöle, Olivenöle, die besonders viel Omega-3-Fettäuren enthalten und native Lecithine, Antioxidantien, Phytohormone, die durch Flavonglycoside antioxidative stabilisert werden. Keimlinge werden als Energielieferanten extrahiert, die Mevalonate, Sterole, Prähormone, Phosphate enthalten.

Besonders für Schwangere oder stillende Mütter werden Folsäure, Jodid, Dehydrocholecalciferole und Mineralien angereichert. Gesunde Fischöle mit Omege-3-Fettsäuren, Tier-, Milch-, Pflanzenprodukte ohne schädliche Mikroben und ohne Schimmel, Hefe werden gewählt und angereichert mit Jodid und Folsäure. Dietätische Kompositionen mit Omega-3-Fettsäuren fördern gesunde Prostaglandine und Prostacycline, um dem Gewichtsanstieg und der endothelialen Dysfunktion zu begegnen die durch Alkoholmetabolite gefördert werden. Alle Kompositionen werden mit Antioxidantien geschützt und werden mit sauren pH-Wert kühl zubereitet und dunkel gelagert. Die Komponenten werden auch mit Kombinationspackungen angeboten, so dass Spurenelemente und Mineralien angereichert werden ohne enzymatische Transformation zwischen den Komponenten z.B. durch Kombiatonspackungen.

Milch oder Molke sind besonders geeignet mit gesundem Albumin, Lipasen, Phospholipasen, Acetylhydrolasen zur reinigenden Pflege vom gesamten Organismus und besonders von Mund, Darm, Haut und vom endothelialen und perivaskulärem System bei sensiblen Personen mit Konsum von Alkohol, Nikotin oder bei Übergewicht, Hyperlipidämien und Leberproblemen, wobei Ziegen- oder Schafmilchprodukte werden verwendet. Milch-, Molke-produkte enthalten natives Serum Albumin, Phospholipasen, Acetylhydrolasen, Antioxidantien, Vitamine und Prähormone und können mit gesundem Kakaogetränken gemischt werden.

Die lokalen, dietätischen Kompositionen werden auch als Kombinationspackung angeboten in nach außen einheitlicher Form, wobei die Produkte mit dem Wortbestandteil Fida^{R} und dem rosa Blatt nach außen einheitlich gekennzeichnet sind. Die Öle eignen sich zur Extraktion und Verdünnung und/der zur Extraktion von Ginkgo- Blättern die mit den Verfahrensschritten der vorliegenden Erfindung geschützt sind. Biozertifizierte Produkte eignen sich erfindungsgemäß für Personen mit kritischen Gewohnheiten, Blutdruckanstieg, Hyperlipidämien, kritischen Blutzuclcerprofilen, zur Gesundheitsförderung und zum Schutz vom Endothelium, von Serum Albumin, oder der HDL-Komponenten. Das unerwartete Problem der exogenen und/oder endogenen Transformation vorzugsweise von Phospholipiden wurde bisher nicht erkannt und wird hier gelöst durch neue Herstellungsverfahren.

Alternde Personen zeigen eine erhöhte Sensibilität durch geschwächte Regenerationsfähigkeit, so dass additive Risikofaktoren besonders schädigen. Sensible Personen mit oxidativem Stress oder Präsenz von Alkoholmetaboliten neigen zum Gewichtsanstieg, Leberfunktionsstörungen und zur endotheliale Dysfunktion. Kleine Gefäße (SMV), geschwächte Drüsen und Potenz und ein Cluster von schädigenden Symptomen sind die Folge mit Anstieg von Blutdruck, Dyslipidämie, niedrigem HDL-Cholesterol, kritischen Blutzuckerprofilen. Diese dyslipidämischen, mikrovaskulären und/oder prädiabetische Syndrome sind im Gebrauchsmuster offenbart und Behandlungen von Personen, die dietätische Nahrungsmittel und alkoholfrei Getränke brauchen, um Organe, Gewebe, Leber, Pankreas, Gehirn, endokrinen Drüsen, Gonaden zu schützen. Neurologische, mentale und perivaskuläre Systeme und Endothelzellen werden geschützt, um der vaskulären Demenz zu begegnen.

Die Haut wird gepflegt, die beim Konsum von Alkohol und Nikotin leidet, degeneriert und/oder mit Lichtschäden reagiert, die hier vermieden werden. Hier werden die Störungen der kleinen Gefäße (SMV) vermieden durch reine, stabile dietätische und lokale Kompositionen. Eine weitere Aufnahme und/oder Bildung von Alkyl-Derivaten wird erfindungsgemäß vermieden durch alkoholfreie Getränke, gesunde Nahrung, viel Flüssigkeit und Bewegung. Anonymisierte, standardisierte klinische Studienprogramme sind anerkannt und genehmigt (BLÄK-EK No. 02088, see Journal Men's Health & Gender, jmhg vol.3, 279-289, 2006).

## Patentansprüche

**1.** Ölige Zubereitung von und mit Lecithinen, **dadurch gekennzeichnet, dass** keine Etherphospholipide gebildet werden bei der Zubereitung von pflegenden, dietätischen Kompositionen und Heilmitteln, die angereichert werden ohne Bildung von Etherphospholipiden.

**2.** Pflegende, dietätische Zubereitung und Kompositionen gemäß Anspruch 1, **dadurch gekennzeichnet, dass** lokale und kosmetische Kompositionen zubereitet werden durch Extraktion von Zellen, Zellfragmenten, Zellprodukten aus dem Pflanzen- und Tierreich, wobei die Bildung von Etherphospholipiden vermindert wird, indem alkylbildende und analoge Stoffwechselwege gehemmt werden, wobei Antioxidantien vorhanden sind.

**3.** Zubereitung gemäß Anspruch 1 oder 2, **dadurch gekennzeichnet, dass** Zellen mit physikalischen Verfahren zerkleinert werden und dass auch Auspressen, Extrahieren und Trennverfahren bei kalten, kühlen Temperaturen durchgeführt werden, um eine enzymatische Bildung von Alkyl-Glycerol, Etherlipiden, Etherphospholipiden, Alkylderivate zu vermindern, die durch alkylbildende und analoge Stoffwechselwege (Alkyl-DHAP-Synthetase und Phosphotransferasen) entstehen in der Zubereitung von Zellen, Zellfragmenten, Zellbestandteilen, Zellprodukten.

**4.** Zubereitung gemäß einem der Ansprüche 1 bis 3, **dadurch gekennzeichnet, dass** dem Verfahren keine Hilfsmittel zugesetzt werden, die Alkylderivate enthalten und dass die Zellfragmente, Zellbestandteile mit ansteigend apolaren Lecithinölen behandelt werden und stufenweise Fettsäuren, Lecithine, und apolare Substanzen angereichert werden, so dass die ansteigend apolaren Kompositionen ohne Löslichkeitsprobleme gemischt werden können mit Milch-, Molkeprodukten.

**5.** Zubereitung gemäß einem der Anspruch 1 bis 4, **dadurch gekennzeichnet, dass** die Kompositionen Lecithine, Omega-3-Fettsäure und Antioxidantien enthalten und mit Serum Albumin, Milch- und Molkeprodukten gereinigt und gemischt werden.

**6.** Zubereitung von Kompositionen gemäss einem der vorstehenden Ansprüche, **dadurch gekennzeichnet, dass** Pflanzenbestandteile ausgewählt werden, die von Beginn kaum Alkylphenole, Pestizide enthalten und keinen Schimmel, keine Bakterien und keine Entzündungsmediatoren (Endotoxine) und keine gerösteteten Produkte verwendet werden, so dass von Beginn keine relevanten Mengen von Alkylglycerol, Etherlipiden, Etherphospholipiden, Alkyl-Derivaten vorhanden sind und diese nicht über Hilfsmittel in das Verfahren eingeführt werden.

**7.** Zubereitung gemäß einem der vorstehenden Ansprüche, **dadurch gekennzeichnet, dass** die gewünschten Kompositionen Lecithine, ungesättigte Fettsäuren, Antioxidantien, Vitamine, Prähormone, Phytohormone, Mineralien, Dehydrocholecalciferole, Spurenlemente, Mevalonate, Sterole, Flavonglycoside enthalten und dass Serum Albumin, Milch-, Molkeprodukten mit Folsäure, Jodid zugefügt wird, wobei die Milch- oder Molkeprodukte Lipasen, Phospholipasen, Acetylhydrolasen enthalten.

**8.** Zubereitung nach einem der vorstehenden Ansprüche **dadurch gekennzeichnet, dass** frische Pflanzenprodukte und auch Fischprodukte aus gesunder Kultur, Zucht verwendet werden für sensible Personen und dass die pflegenden, lokalen, dietätischen Kompositionen keine schädigenden Fette, Salze, Carbohydrate enthalten und keinen Alkohol, wobei Fischöle mit Omega-3-Fettsäuren mit Lecithinen, Antioxidantien, Vitaminen, Prähormonen, Mineralien, Calcium Jodid, Folsäure, Dehydrocholesterolen nach Bedarf angereichert werden.

**9.** Örtliche und dietätische Kompositionen nach einem der Ansprüche 1 bis 8 **dadurch gekennzeichnet, dass** Serum Albumin, fettarme Milch-, Molkeprodukten, Dehydrocholesterol, Antioxidantien, Vitamine, Mineralien, Enzyme enthalten, besonders Lipasen, Phospholipasen, Acetylhydrolasen, die angereichert oder zugefügt werden, um Alkohol zu ersetzen, zu neutralisieren, abzubauen, wobei von Beginn keine Schimmelpilze oder Entzündungsmediatoren (Endotoxine) enthalten in Komposition, die **gekennzeichnet sind durch** den nachweislichen Ausschluss von Etherlipiden, Ether Phospholipiden, Alkyl-Derivaten für eine reinigenden Pflege von Haut, Mund, Darm und Haut und dass Etherphospholipide im Mund und Darm abgebaut werden.

**10.** Lokale, kosmetische, dietätische Kompositionen nach einem der Ansprüche 1 bis 9, **gekennzeichnet durch** die Zubereitung von gesunden Keimlingen, Kernen, Nüssen, Samen, die acetylierende Substanzen und energiereiche Phosphate enthalten oder **durch** Zubereitung von Pflanzenölen aus der Gruppe der Keimöle, Kernöle, Rapsöle, Palmöle, Sojaöle, Leindotteröle, Kürbiskernöle, Sonnenblumenöle, Hanföle, Leinöle, Olivenöle, Nussöle, Speiseölen, die ausgewählt, angereichert, gemischt werden und keine Etherphospholipide enthalten.

**11.** Lokale, dietätische Kompositionen nach einem der Ansprüche 1 bis 10, **dadurch gekennzeichnet, dass** von Beginn Mikroben, Schimmel, Hefe, Pestiziden ausgeschlossen sind bei der Zubereitung von Fischölen, Pflanzenölen, Milchprodukten und dass gesunde Komponenten unverändert zubereitet werden, die Lecithine, Omega-3-Fettsäuren, antioxidative Flavonoide, Serum Albumin enthalten, wobei Dehydrocholesterole, Mineralien,
Jodid, Folsäure, Vitamine, Spurenelemente, energiereiche Substanzen, angereichert oder zugefügt werden besonders für schwangere Frauen, stillende Mütter, Kinder und zum Schutz vom Endothel, der Blutkompartimente, der Gewebe, der Organe der Haut, des Darms, der Leber, der Drüsen von sensiblen Personen.

**12.** Kosmetische, pflegende Kompositionen nach einem der Ansprüche 1 bis 11, die durch den Ausschluss von Etherphospholipiden gekennzeichnet sind und unveränderte Komponenten enthalten, nämlich gesunde Lecithine, Omega-3-Fettsäuren, Ceramide, Vitamine, antioxidative Substanzen oder Serum Albumin mit Mineralien, Sterolen, Xanthinen angereichert werden, wobei die gewünschten Komponenten auch zugefügt werden können.

**13.** Dietätische Kompositionen nach einem der Ansprüche 1 bis 12, die durch Omega-3-Fettsäuren gekennzeichnet sind, um gesunde Prostaglandine, Prostacycline zu erhöhen und damit der endothelialen Dysfunktion durch Alkoholmetabolite zu begegnen, wobei die Komponnten und Kompositionen mit Antioxidantien geschützt werden.

**14.** Kompositionen nach einem der Ansprüche 1 bis 13, **dadurch gekennzeichnet, daß** ein oder mehrere Verfahrensschritte oder Komponenten einen sauren pH-Wert haben oder dass die Komponenten mit Kombinationspackungen angeboten werden, die Milch-, Molkeprodukte mit und Mineralien trennen von Lecithinen und Spurenelementen um eine späte alkylartige Transformation zu hemmen oder dass die Kompositionen kühl und abgedunkelt gelagert werden.

**15.** Lokale, dietätische Kompositionen nach einem der Ansprüche 1 bis 14, **dadurch gekennzeichnet, dass** die öligen Komponenten mit einer schadstoffarmen Nahrung, alkoholfreien Getränken angeboten werden, die keine schädigenden Fetten, Salze, Carbohydrate enthalten, um endogene Alkoholmetabolite auszugleichen, indem Milch-, Molkeprodukte Phospholipasen, Acetylhydrolasen enthalten sind, die Alkoholmetabolite schon im Darm abbauen, wobei besonders Ziegen- oder Schafsmilchprodukte schonend zubereitet, angereichert wird.

**17.** Kompositionen nach einem der Ansprüche 1 bis 16, **dadurch gekennzeichnet, dass** möglichst reines Serum Albumin mit alkoholfreien Getränken zubereitet wird, wobei Milch- oder Molkeprodukte verwendet werden, die gesunde Lipasen, Phospholipasen, Acetylhydrolasen enthalten zur Pflege, Reinigung vom gesamten Organismus und besonders von Mund, Darm, Haut und vom endothelialen und perivaskulärem System oder beim Konsum von Alkohol, Nikotin oder Gewichtsproblemen, Hyperlipidämien, bei Leberproblemen von sensiblen Personen.

**18.** Lokale, dietätische Kompositionen nach einem der Ansprüche 1 bis 17, wobei Ziegen- oder Schafmilchprodukte verwendet werden, die natives Serum Albumin, Phospholipasen, Acetylhydrolasen, Antioxidantien, Vitamine und Prähormone enthalten und angeboten werden als Kombinationspackung mit ölig zubereiteten Ginkgoloiden.

**19.** Lokale, kosmetische, dietätische Kompositionen nach einem der Ansprüche 1 bis 18, **dadurch gekennzeichnet, dass** Lecithine zur Zubereitung von öligen Extrakten der Ginkgoblätter verwendet werden und dass Ginkgoloidöle mit Pflanzenölen, Pflanzenbutter gemischt werden zur Zubereitung von milchigen, cremigen Kompositionen.

**20.** Ölige Zubereitungen nach dem Anspruch 19, **dadurch gekennzeichnet, dass** die Ginkgolidöle milchig oder cremig gelöst werden und Flavonoide und aktive Ginkgolide enthalten.
